# FASCICULE DE BREVET EUROPEEN

(11) **EP 0 986 376 B1**
(45) Date de publication et mention de la délivrance du brevet: **23.01.2008**
(21) Numéro de dépôt: 98929521.7
(22) Date de dépôt: 09.06.1998
(51) Int. Cl.: A61K 31/454, A61K 31/702, A61P 7/02

(54) **Utilisation d'un oligosaccharide INHIBITEUR DU FACTEUR Xa EN COMBINAISON AVEC UN ANTIAGREGANT PLAQUETTAIRE pour le traitement de LA THROMBOSE ARTERIELLE**
VERWENDUNG VON einem Faktor a inhibierenden Oligosacchariden MIT EINEM BLUTGERINNUNGSHEMMENDEN MITTEL ZUR BEHANDLUNG VON ARTERIENTHROMBOSEN
USE OF A FACTOR Xa INHIBITing oligosaccharide WITH A PLATELET ANTIAGGREGATING AGENT FOR TREATING ARTERIAL THROMBOSIS

(30) Priorité: 13.06.1997 FR 9707368
(43) Date de publication de la demande: 22.03.2000
(73) Titulaire: Sanofi-Aventis, 75013 Paris (FR)
(72) Inventeur: BERNAT, André, F-31270 Cugnaux (FR); HERBERT, Jean-Marc, F-31170 Tournefeuille (FR); PETITOU, Maurice, F-75645 Paris Cedex 13 (FR); VAN AMSTERDAM, Ronald, NL-5384 BJ Heesch (NL)
(74) Mandataire: Thouret-Lemaitre, Elisabeth
(86) Numéro de dépôt international: PCT/FR1998/001172
(87) Numéro de publication internationale: WO 1998/056365

(56) Documents cités:
- EP-A- 0 138 632
- EP-A- 0 540 051
- A. VUILLEMENOT ET AL.: "SUSTAINED ANTI-THROMBOTIC ACTIVITY OF A SULPHATED PENTASACCHARIDE, A PURE ANTI FACTOR-Xa INHIBITOR ADMINISTERED AS UNIQUE ANTICOAGULANT IN CORONARY ANGIOPLASTY" EUROPEAN HEART JOURNAL, vol. 18, août 1997, page 622 XP002078330
- B. KAISER ET AL.: "GLYCOPROTEIN IIb/IIIa INHIBITORS POTENTIATE THE INHBIBITION OF THROMBIN GENERATION BY FACTOR Xa BUT NOT BY THROMBIN INHIBITORS" BLOOD, vol. 90, no. 10, 15 novembre 1997, page 97B XP002078331
- HERBERT ET AL.: "DX 9065A, a novel, synthetic, selective and orally active inhibitor of Factor Xa: in vitro and in vivo studies" J. PHARMACOL. EXP. THER., vol. 276, no. 3, mars 1996, pages 1030-1038, XP002057652
- KUNITADA ET AL.: "Factor Xa inhibitors" CURR. PHARM. DESIGN, vol. 2, no. 5, octobre 1996, pages 531-542, XP002057653
- SCHIELE F J ET AL: "INITIAL EXPERIENCE OF A SULPHATED PENTASACCHARIDE, A PURE FACTOR XA INHIBITOR, IN CORONARY ANGIOPLASTY" CIRCULATION, vol. 94, no. 8, 15 octobre 1996, XP002046076
- HERBERT ET AL.: "SR90107A/Org 31540, a novel anti-factor Xa antithrombotic agent" CARDIOVASC. DRUG REV., vol. 15, no. 1, 21 mars 1997, pages 1-26, XP002057654 (SPRING)
- CADROY Y ET AL: "ANTITHROMBOTIC EFFECTS OF SYNTHETIC PENTASACCHARIDE WITH HIGH AFFINITY FOR PLASMA ANTITHROMBIN III IN NON-HUMAN PRIMATES" THROMBOSIS AND HAEMOSTASIS, vol. 70, no. 4, pages 631-635, XP002046072
- ZANDBERG ET AL.: "Effect of the synthetic pentasaccharide ORG 31540/SR90107 and heparin on thrombolysis and reocclusion after successful lysis of a thrombus in the femoral artery of a rabbit" FIBRINOLYSIS, vol. 10, no. suppl3, 24 - 28 juin 1996, page 83 XP002057655
- VAN AMSTERDAM ET AL.: "Evaluation of ORG 31540/SR 90107A in preclinical models of thrombosis" THROMB. HAEMOST., vol. suupl., 6 - 12 juin 1997, page 2834 XP002057656
- YAMASHITA ET AL.: "The antithrombotic effect of synthetic low molecular weight human factor Xa inhibitor, DX-9065A, on HE-NE laser-induced thrombosis in rat mesenteric microvessels" THROMB. RES., vol. 85, no. 1, janvier 1997, pages 45-51, XP002057657
- KAISER ET AL.: "Factor Xa versus Factor IIa inhibitors" CLIN. APPL. THROMB. HEMOST., vol. 3, no. 1, janvier 1997, pages 16-24, XP002057658
- FUKUDA ET AL.: "Beneficial effect of DX-9065A, a selective factor Xa inhibitor, in a ferric chloride-induced arterial thrombosis model in rats" JPN J. PHARMACOL., vol. 71, no. sup.1, 1996, page 327p XP002057659
- VOGEL ET AL.: "Two new closely related rat models with relevance to arterial thrombosis - Efficacies of different antithrombotic drugs" THROMB. HAEMOST., vol. 77, no. 1, janvier 1997, pages 183-189, XP002057660
- HERBERT ET AL.: "Biochemical and pharmacological properties of SANORG 32701: comparison with the "synthetic pentasaccharide" (SR 90107/ ORG 31540) and standard heparin" CIRC. RES., vol. 79, no. 3, septembre 1996, pages 590-600, XP002057661
- VERSTRAETE ET AL.: "Novel antithrombotic drugs in development" DRUGS, vol. 49, no. 6, 1995, pages 856-884, XP002057662
- BERNAT ET AL.: "The syntheric pentasaccharide SR 90107A/Org 31540 enhances tissue-type plasminogen activator-induced thrombolysis in rabbits" FIBRINOLYSIS, vol. 10, no. 3, 1996, pages 151-157, XP002046074
- FITZGERALD ET AL.: "Specific glycoprotein IIb/IIIa antagonists: will they be an improvement on apsirin in atherogenic cardiovascular disease ?" EXPERT OPIN. THER. PATENTS, vol. 5, no. 11, 1995, pages 1143-1146, XP002057666
- HERAULT ET AL.: "In vitro inhibtion of heparin-induced platelet aggregation in plasma from patients with HIT by SR 121566, a newly developed Gp IIb/IIIa antagonist" BLOOD COAGUL. FIBRINOLYSIS, vol. 8, no. 3, avril 1997, pages 206-207, XP002057667
- SAVI ET AL.: "SR 121787: a potent, synthetic and orally active platelet fibrinogen receptor antagonist" THROMB. HAEMOST., vol. suppl, juin 1997, page 1599 XP002057668

## Description

La présente invention a pour objet l'utilisation d'inhibiteurs sélectifs indirects du facteur Xa agissant via l'antithrombine III, en combinaison avec un composé à activité antiagrégante plaquettaire, pour leur activité dans les maladies thromboemboliques artérielles.

Les compositions pharmaceutiques contenant l'association des principes actifs antithrombotiques et antiagrégants plaquettaires font également partie de l'invention.

Les principes actifs constituant l'association sont présents à l'état libre ou sous forme d'un de leurs sels pharmaceutiquement acceptable.

Au cours de cette dernière décennie, un grand intérêt a été accordé à l'étude du rôle joué par les plaquettes dans le développement des maladies associées à l'athérosclérose (infarctus du myocarde, angor, accident vasculaire cérébral, maladies artérielles des membres inférieurs....). D'autre part, le rôle bien établi du processus de la coagulation sanguine dans la thrombose artérielle a permis le développement de nombreux médicaments qui inhibent les diverses enzymes de la coagulation. La découverte du rôle essentiel de la thrombine et du facteur Xa dans le processus thrombotique a conduit à proposer l'utilisation des anticoagulants dans la prévention et le traitement de la thrombose artérielle.

L'efficacité du DX9065A, un inhibiteur direct du facteur Xa, dans le traitement des thromboses est décrit dans J. Pharmacol. Exp. Ther, vol, 276, n° 3, 1996, pp 1030 - 1038.

L'activité antithrombotique du SR 901071/Org 31540, un inhibiteur sélectif du facteur Xa, est décrit 'Cardiovascular Drugs Reviews, vol 15 n 1, pp 1-26; Cric. Research, vol. 79, n° 3, 1996, pp 590-600) mais ce composé est administré comme seul principe actif. L'utilisation d'une injection simple de ce composé SR 901071/Org 31540 en bolus et de l'aspirine par voie i.v. lors d'une angioplastie coronarienne est décrite (Circulation, vol. 96, n° 8, 1996/5).

Parmi les anticoagulants disponibles l'héparine est le médicament privilégié dans la prévention et le traitement des maladies thromboemboliques.

L'héparine catalyse notamment via l'anfithrombine III (AT III) l'inhibition de deux enzymes qui interviennent dans la cascade de la coagulation du sang à savoir, le facteur Xa et le facteur IIa (ou thrombine). L'importance relative de ces deux activités dans l'activité globale de l'héparine reste inconnue. Les préparations d'héparines de bas poids moléculaire (HBPM), contiennent des chaînes formées de 4 à 30 monosaccharides qui agissent comme l'héparine sur le facteur Xa et sur la thrombine mais qui ont la propriété d'être plus sélectives du facteur Xa que de la thrombine.

Malgré ce profil d'activité biologique différente, l'héparine de bas poids moléculaire a un effet antithrombotique comme cela a été mis en évidence dans des études sur les animaux et sur des patients souffrant de maladies thromboemboliques ou présentant des risques de formation d'un thrombus (Hirsch J. et al, J. Thromb. Hemost., 1987, Leuven, Belgium Leuven University Press, 325-348).

A la différence de l'héparine et des HBPM, certains oligosaccharides de synthèse notamment ceux décrits dans EP 84999 ont la propriété d'inhiber sélectivement, via l'antithrombine III, le facteur Xa mais ne possèdent aucune activité sur la thrombine.

Ces oligosaccharides de synthèse correspondant au domaine de liaison à l'antithrombine (DLA) de l'héparine sont connus et manifestent une activité antithrombotique dans la thrombose veineuse. Ces composés sont décrits dans EP 529715, EP 621282.

L'efficacité de ces oligosaccharides dans la prévention de la thrombose artérielle était peu vraisemblable du fait de leur incapacité à inhiber la thrombine.

En effet, il est connu de longue date dans la littérature que la thrombine joue un rôle clé dans la thrombose artérielle et ceci est encore confirmé par des expériences récentes (L. A. Harker, Blood, 1991, 77, 1006-1012). Les inhibiteurs de thrombine, constituent donc un moyen efficace de prévenir et combattre ce type de thrombose.

On a constaté, en comparant l'efficacité de l'héparine à celles des inhibiteurs directs de thrombine (on appelle inhibiteur direct un inhibiteur qui inhibe la thrombine sans passer par l'intermédiaire de l'AT III) que ces derniers sont beaucoup plus efficaces que l'héparine pour prévenir et traiter la thrombose artérielle (Arteriosclerosis and thrombosis, 1992, 12, 879-885. J. Am. Coll. Cardiol., 1994, 23, 993-1003). La raison de ce manque d'efficacité est que le complexe héparine/AT III ne peut, pour des raisons d'encombrement stérique, inhiber la thrombine dans un thrombus riche en plaquettes comme l'est un thrombus plaquettaire.

La faible activité de l'héparine par opposition aux inhibiteurs directs est donc liée à son besoin d'utiliser l'AT III. Cette explication est encore justifiée par l'observation récente que les inhibiteurs directs du facteur Xa agissant sans AT III sont également efficaces, dans des modèles animaux de thrombose artérielle (Circulation, 1991, 84, 1741-1748 Thrombosis Haemost., 1995, 74, 640-645).

On s'attendrait donc à ce qu'un composé qui, d'une part agit par l'intermédiaire de l'AT III, et d'autre part n'inhibe pas la thrombine, ne possède aucune activité en thrombose artérielle.

Le document Drugs vol 49 n° 6 1995, pp 856-884, traite des nombreux inconvénients des médicaments antithrombotiques connus et mentionne seulement comme voie de recherche l'association d'un antiagrégant soit avec un anticoagulant inhibiteur spécifique de la thrombine, soit avec un inhibiteur du facteur Xa, mais non leur combinaison.

On a maintenant trouvé, selon la présente invention, d'une façon tout à fait surprenante qu'un d'inhibiteur sélectif indirect du facteur Xa, en combinaison avec un composé à activité antiagrégante plaquettaire peut être utilisé pour leur activité dans les maladies thromboemboliques d'origine artérielle.

Bien qu'il soit connu, à ce jour, que les anti-facteurs Xa et les antiagrégants plaquettaires agissent *via* deux mécanismes d'action différents, la combinaison ou l'association de ces produits pour une utilisation dans les maladies thromboemboliques artérielles n'a jamais été étudiée.

Ainsi, la présente invention a pour objet, selon un de ses aspects, l'utilisation d'un ou plusieurs inhibiteurs sélectifs indirects du facteur Xa en combinaison avec un ou plusieurs composés à activité antiagrégante plaquettaire, pour la préparation de médicaments destinés à prévenir et à traiter les maladies thromboemboliques d'origine artérielle.

Selon l'invention, on entend par inhibiteur sélectif du facteur Xa un composé capable d'inhiber sélectivement le facteur Xa via l'antithrombine (AT III) mais ne présentant pas d'activité significative vis-à-vis de la thrombine. De préférence, l'inhibiteur n'a aucune activité vis-à-vis de la thrombine.

Se!on un autre de ses aspects la présente invention a également pour objet, l'utilisation d'un inhibiteur sélectif du facteur Xa agissant via l'AT III, en combinaison avec un composé à activité antiagrégante plaquettaire, pour la préparation de médicaments destinés à combattre les maladies thromboemboliques d'origine artérielle.

Parmi les inhibiteurs sélectifs indirects du facteur Xa et de façon avantageuse, les oligosaccharides de synthèse sont des pentasaccharides, tels que ceux revendiqués dans les brevets EP 84999 et US 5,378,829.

Des pentasaccharides particulièrement avantageux sont notamment le : Méthyl O-(2-déoxy-2-sulfoamino-6-O-sulfo-α-D-glucopyranosyl)-(1→4)-O-(acide β-D-glucopyranosyluronique)-(1→4)-O-(2-déoxy-2-sulfoamino-3,6-di-O-sulfo-α-D-glucopyranosyl-(1→4)-O-(acide 2-O-sulfo-α-L-idopyranosyluronique)-(1→4)-2-déoxy-2-sulfoamino-6-O-sulpho-α-D-glucopyranoside dont l'anion a la structure (B) 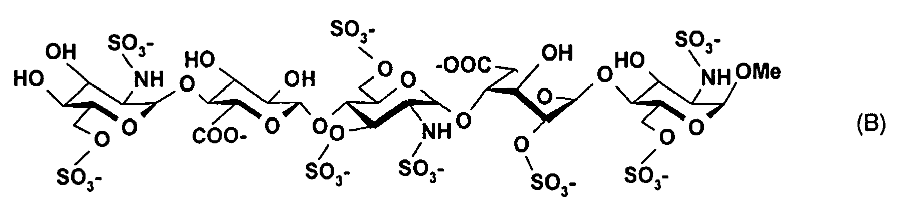 et ses sels pharmaceutiquement acceptables, notamment son sel décasodique, connu sous son nom de code SR 90107 / ORG 31540, décrit dans Chemical Synthesis to Glycoaminoglycans, Supplement to Nature 1991, 350, 30-33, ci-après désigné "PS" .

Le méthyl O-(3,4-di-O-méthyl-2,6-di-O-sulfo-α-D-glucopyranosyl)-(1→4)-O-(acide 3-O-méthyl-2-O-sulfo-β-D-glucopyranosyluronique)-(1→4)-O-(2,3,6-tri-O-sulfo-α-D-glucopyranosyl)-(1→4)-O-(acide 3-O-méthyl-2-O-sulfo-α-L-idopyranosyluronique)- (1→4)-2,3,6-tri-O-sulfo-α-D-glucopyranoside, connu sous son nom de code SANORG 32701, dont l'anion a la structure (C) 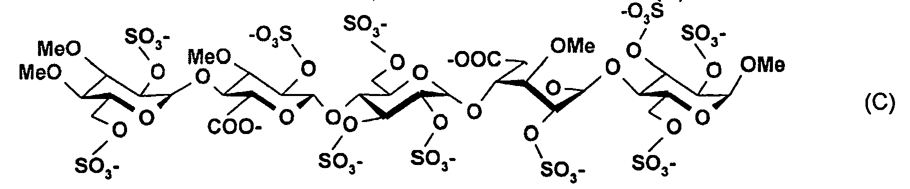 et ses sels pharmaceutiquement acceptables, notamment son sel de dodécasodium, décrit dans US 5,378,829 ;

Le méthyl O-(2,3,4-tri-O-méthyl-6-O-suffo-α-D-glucopyranosyl)-(1→4)-O-(acide 2,3-di-O-méthyl-β-D-glucopyranosyluronique)-(1→4)-O-(2,3,6-tri-O-sulfo-α-D-glucopyranosyl)-(1→4)-O-(acide 2,3-di-O-méthyl-α-L-idopyranosyluronique)-(1→4)-O-2,3,6-tri-O-sulfo-α-D-glucopyranoside connu sous son nom de code SANORG 34006 dont l'anion a la structure (D) 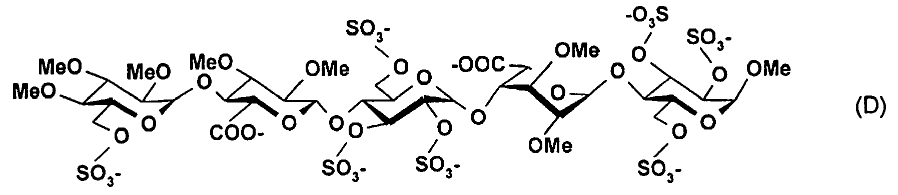 et ses sels pharmaceutiquement acceptables, notamment son sel de nonasodium, également décrit dans US 5,378,829.

Le composé de formule (B), de préférence sous forme de sel décasodique fait l'objet d'une utilisation préférée selon l'invention et fait également l'objet de l'antithrombotique préféré des compositions pharmaceutiques de l'invention en association avec un antiagrégant plaquettaire. Les antiagrégants plaquettaires sont avantageusement choisis parmi le groupe XXXVIII ci-après :

XXXVIII - Le SR 121787A ou une de ses analogues de formule: 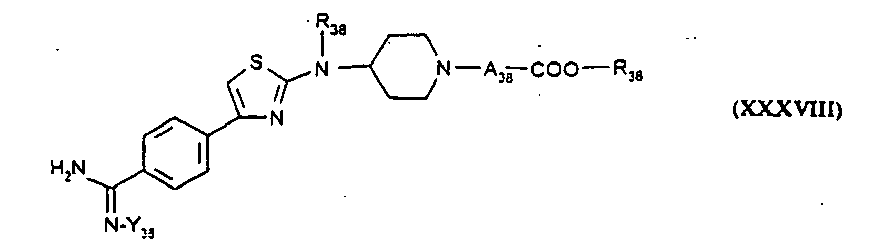 dans laquelle :
- R₃₈¹ représente l'hydrogène, un alkyle en C₁-C₅, un cycloalkyle en C₃-C₈: un aralkyle dont la partie alkyle est en C₁-C₅, un groupe alcoxycarbonylakyle dans lequel les parties alcoxy et alkyle sont en C₁-C₃ ou un groupe carboxyalkyle dans lequel la partie alkyle est en C₁-C₃ ;
- A₃₈ représente soit un groupe méthylène éventuellement mono ou di-substitué par un groupe alkyle en C₁-C₅ ; par un groupe alcoxycarbonyle dont la partie alcoxy est en C₁-C₅ ; par un groupe alcoxycarbonylalkyle dans lequel les parties alcoxy et alkyle sont en C₁-C₅ ; par un groupe carboxyalkyle dont la partie alkyle est en C₁-C₅ ; par un groupe phényle ou benzyle non substitué ou substitué sur le noyau aromatique par un alkyle en C₁-C₅, un alcoxy en C₁-C₅, un hydroxyle, un halogène ou un trifluorométhyle; par un groupe pyridyle; soit un groupe éthylène;
- R₃₈ représente l'hydrogène, ou un groupe alkyle en C₁-C₅; un groupe aryle ou un groupe aralkyle dont la partie alkyle est en C₁-C₅ éventuellement substitué par un hydroxyle, un alcoxy en C₁-C₃, un halogène, un trifluorométhyle ou un alkyle en C₁-C₅:

- Y₃₈ représente l'hydrogène : un groupe -COOR₃₈² dans lequel R₃₈² représente un groupe alkyle en C₁-C₅, un groupe aryle ou un groupe aralkyle dont la partie alkyle est en C₁-C₅ éventuellement substitué par un alkyle en C₁-C₅; un groupe
- COR₃₈³ dans lequel R₃₈³ représente un alkyle en C₁-C₅; ou un de leurs sels, tels que décrit dans EP 719775.
   dans laquelle :

Les antiagrégants plaquettaires utilisés sont les antagonistes de la glycoprotéine IIb/IIIa choisis parmi:
   - le N-(1-éthoxycarbonylméthyl-pipéridin-4-yl)-N-{4-[4-{(N-éthoxycarbonylimino)(amino)-méthyl}-phényl]-thiazol-2-yl}-3-aminopropionate d'éthyle, désigné SR 121787A, et ses sels pharmaceutiquement acceptables, et
   - l'acide N-(1-carboxyméthyl-pipéridin-4-yl)-N-{4-[4-{(amino)(imino)méthyl}-phényl]-thiazol-2-yl}-3-aminopropionique,
   et ses sels pharmaceutiquement acceptables, tels que le trichlorhydrate, désigné SR 121566.
Les formules du SR 121787 et du SR 121566 sont rappelées ci-dessous :

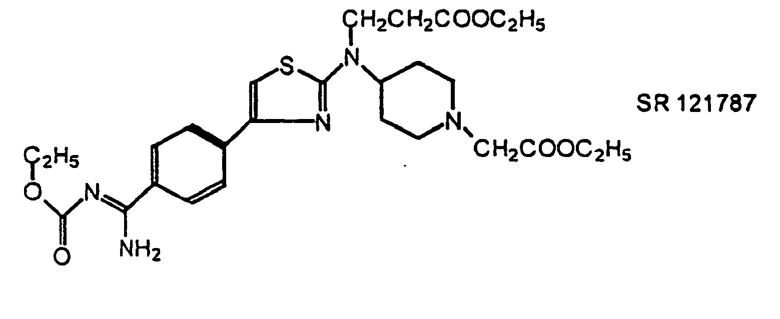

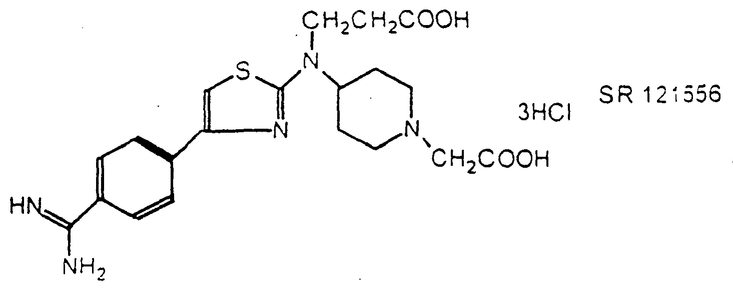

L'inhibiteur sélectif du facteur Xa, combinaison avec un antiagrégant plaquettaire, est particulièrement utile pour le traitement ou la prophylaxie des états pathologiques tels que les troubles du système cardiovasculaire et cérébrovasculaire comme les troubles tromboemboliques associés à l'athérosclérose ou au diabète tels l'angine instable, l'attaque cérébrale, la resténose après l'angioplastie, l'endartérectomie ou pose de prothèses endovasculaires métalliques, ou comme les troubles thromboemboliques associés à la rethrombose après thrombolyse, à l'infarctus, à la démence d'origine ischémique, aux maladies artérielles périphériques, à l'hémodialyse, aux fibrillations auriculaires, ou encore lors de l'utilisation de prothèses vasculaires, de pontages aorto-coronariens ou vis à vis de l'angor stable ou instable, ou encore, *vis* à *vis* de patients traités par une procédure de revascularisation à risques de thrombose incluant les angioplasties percutanées, les prothèses endovasculaires, les prothèses vasculaires, les pontages aorto-coronariens.

Comme exemples de sels organiques pharmaceutiquement acceptables, on peut citer les oxalate, maléate, fumarate, méthanesulfonate, benzoate, ascorbate, pamoate, succinate, hexamate, bisméthylènesalicylate, éthanedisuifonate, acétate, propionate, tartrate, salicylate, citrate, gluconate, lactate, malate, cinnamate, mandélate, citraconate, aspargate, palmitate, stéarate, itaconate, glycolate, p-aminobenzoate, glutamate, benzènesulfonate et théophylline acétate ainsi que les sels formés à partir d'un acide acide aminé tel que le sel de lysine ou d'histidine.

Comme exemples de sels inorganiques pharmaceutiquement acceptables, on peut citer les chlorhydrate, bromhydrate, sulfate, sulfamate, phosphate et nitrate.

La neutralisation du facteur X activé (Xa) (par l'antithrombine III) peut être catalysée par le pentasaccharide (PS) comme par l'héparine de bas poids moléculaire. Contrairement à l'héparine de bas poids moléculaire, le pentasacchande est totalement dénué d'activité sur la thrombine. En outre, le pentasaccharide modifie peu les tests de l'hémostase et notamment le test "du temps de céphaline activée" ou "activated partial thromboplastin time" (APTT) sur du plasma humain et contrairement à l'héparine, il ne potentialise pas l'ADP ou l'agrégation plaquettaire induite par le collagène.

Néanmoins, les effets antithrombotiques du pentasaccharide ont été démontrés après administration intraveineuse (i.v.) et sous cutanée (s.c.) dans différents modèles animaux avec différents types de thromboses induites (le modèle de stase veineuse chez le rat, le modèle de shunt artério-veineux chez le rat, le modsle de Wessler chez le lapin) (Hobellen PMJ et al, Tromb. & Haemost., 1996, 63(2), 265-270 et Amar J. et al, 8r. J. Haematol., 1990, 76, 94-100). En ce qui concerne le risque de saignement, la perte de sang à forte dose de PS testée sur des rats (21,7 mg/kg) est identique à celle constatée avec 200 unités anti Xa/kg d'héparine, indiquant ainsi que le PS n'accroit que peu la perte de sang comparativement à l'héparine standard ou les HBPM.

Afin d'illustrer l'invention et à titre d'exemple, on donnera ci-après les résultats obtenus lors de l'étude de la potentialisation de l'effet antithrombotique chez le lapin par la co-administration d'un inhibiteur sélectif du facteur Xa et d'un antiagrégant plaquettaire. En tant qu'inhibiteur sélectif du facteur Xa le SR 90107 / ORG 31540 (ou PS) a été choisi et l'antiagrégant plaquettaire a été choisi parmi les antagonistes de la glycoprotéine IIb/IIIa, à savoir le SR 121787A.

Le but de cet essai était donc de déterminer les effets antithrombotiques artériels de la co-administration d'un anti Xa, le pentasaccharide SR 90107 / ORG 31540 et d'un antagoniste des récepteurs GPIIb/IIIa, SR 121787A.

Des lapins mâles New-Zealand pesant 2,7 à 3 kg provenant de l'élevage Lago (France) ont été utilisés. Ils étaient hébergés dans des conditions standard.

La formation du thrombus était induite par la stimulation électrique externe de la carotide commune gauche selon la méthode de Hladovec I. et al. (Experimental arterial thrombosis in rats with continuous registration. Thromb. Diathes. Haemor. 1971; 26 : 407-410). Les lapins étaient anesthésiés avec du pentobarbital à la dose de 30 mg/kg iv. Un segment de l'artère carotide gauche était exposé et isolé par un morceau de film isolant. Des électrodes étaient insérées sous l'artère. Une striction partielle induisant une réduction de 20% du débit était réalisée sur l'artère. Celle-ci était alors stimulée à l'aide d'un courant de 2,5 mA délivré par un stimulateur à courant constant pendant 3 minutes. L'occlusion thrombotique était évaluée par la mesure continue du débit du sang dans la carotide par l'intermédiaire d'un débimètre électromagnétique NARCO. Cette mesure était réalisée pendant une période d'observation de 45 minutes.

Pour le traitement, des solutions de SR 90107 / ORG 31540 et de SR 121787A étaient préparées extemporanément dans une solution saline. Les solutions de SR 90107 / ORG 31540 étaient injectées par voie iv 5 minutes avant l'induction de la thrombose dans un volume de 1 ml/kg. Le SR 121787A était administrée par voie orale 2 heures auparavant.

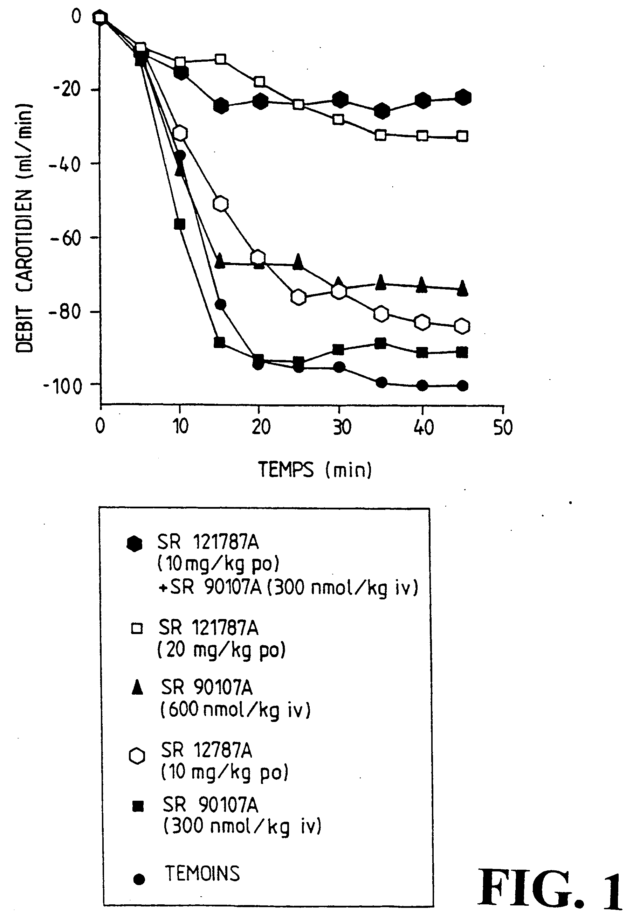

Les résultats étaient calculés et exprimés comme le % de réduction du débit sanguin à divers temps par rapport au temps 0. Ils sont regroupés sur la figure 1.

Chez les animaux témoins, le débit était progressivement réduit de 20.9±2,1 à 3.8±2,5 ml/min (n = 8 : moyenne pour 8 animaux) en 15 minutes et 1.5±1.4 ml/min en 20 min. Il se maintenait ensuite à ce niveau jusqu'à 45 minutes. Le SR 90107/ORG 31540, à la dose de 300 nmol/kg, n'avait aucune influence sur cette diminution de débit. 600 nmol/kg de SR 90107 / ORG 31540 la réduisait légèrement mais non significativement.

SR 121787A administré 2 heures avant en tant que seul principe actif, à la dose orale de 10 mg/kg ne modifiait pas significativement l'évolution du débit carotidien tandis que 20 mg/kg inhibait presque totalement la chute de ce dernier.

La co-administration du pentasaccharide SR 90107 /ORG 31540 et de l'antagoniste du récepteur GPIIb/IIIa le SR 121787A, à des doses séparément inactives (respectivement 300 nmol/kg iv et 10 mg/kg po), a complètement protégé contre la réduction du débit et donc contre la thrombose artérielle ainsi induite.

Aucun effet toxicologique du SR 90107 / ORG 31540 n'a été observé sur quelque espèce animale que ce soit, et quelle que soit la concentration testée dans les études de toxicité appropriées et par dosages répétés (sur une période de 4 semaines avec jusqu'à 10 mg/kg sous cutanés). Le SR 90107 / ORG 31540 n'est pas mutagène dans le test de AMES, ou dans le test de réparation de l'ADN.

Ainsi l'utilisation d'un inhibiteur sélectif indirect du facteur Xa, tel qu'un oligosaccharide, en combinaison avec un antiagrégant plaquettaire est particulièrement bénéfique *vis à vis* d'états pathologiques tels que les troubles du système cardiovasculaire et cérébrovasculaire comme les troubles thromboemboliques associés à l'athérosclérose ou au diabète tels l'angine instable, l'attaque cérébrale, la resténose après angioplastie, l'endartérectomie ou pose de prothèses endovasculaires métalliques ou comme les troubles thromboemboliques associés à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires ou de pontages aorto coronariens, ou, *vis à vis* de l'angor stable ou instable, ou encore *vis* à *vis* de patients traités par une procédure de revascularisation a risques de thrombose incluant les angioplasties percutanées, les prothèses endovasculaires, les prothèses vasculaires, les pontages aorto-coronariens.

En outre, l'utilisation d'un inhibiteur sélectif indirect du facteur Xa, tel qu'un oligosaccharide, en association avec les antiagrégants plaquettaires, ne majore pas le risque hémorragique.

L'association d'un inhibiteur sélectif indirect du facteur Xa, tel qu'un oligosaccharide et de l'antiagrégant plaquettaire peut être formulée dans les compositions pharmaceutiques utilisables par voie orale ou parentérale, notamment par voie sous cutanée, en mélange avec des excipients pharmaceutiques classiques.

Ainsi, selon un autre de ses aspects, l'invention concerne les compositions pharmaceutiques comprenant un ou plusieurs inhibiteurs sélectifs indirects du facteur Xa agissant via l'antithrombine III en association avec un ou plusieurs composés à activité antiagrégante plaquettaire, et, éventuellement un ou plusieurs véhicules pharmaceutiquement acceptables.

Les compositions pharmaceutiques préférées de l'invention comprennent un antagoniste de la glycoprotéine IIb/IIIa en tant qu'antiagrégant plaquettaire et le méthyl O-(2-déoxy-2-sulfoamino-6-O-sulfo-α-D-glucopyranosyl)-(1→4)-O-(acide β-D-glucopyranosyluronique)-(1→4)-O-(2-déoxy-2-sulfoamino-3,6-di-O-sulfo-α-D-glucopyranosyl-(1→4)-O-(acide 2-O-sulfo-α-L-idopyranosyluronique)-(1→4)-2-déoxy-2-sulfoamino-6-O-sulpho-α-D-glucopyranoside dont l'anion a la structure (B) 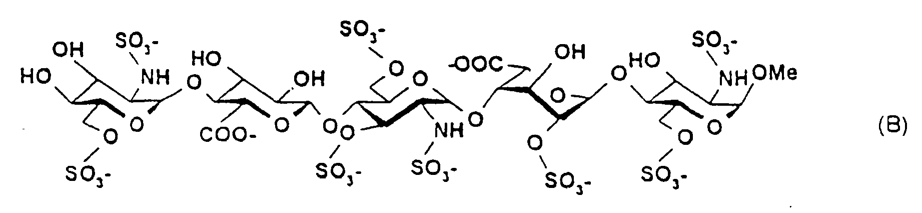 ou l'un de ses sels pharmaceutiquement acceptables, en tant qu'inhibiteur sélectif indirect du facteur Xa.

Les compositions pharmaceutiques, objet de la présente invention, se présentent de préférence sous forme d'unités de dosage contenant une quantité prédéterminée des principes actifs, telle que précisée ci-dessous. Les formes unitaires d'administration par voie orale comprennent les comprimés, les gélules, les poudres, les granules, les microgranules.

L'antiagrégant plaquettaire peut être formulé selon des méthodes bien connues de l'homme de l'art. Il en est de même pour l'inhibiteur sélectif du facteur Xa.

Dans la formulation des associations des principes actifs pour la préparation des compositions pharmaceutiques selon la présente invention, on tiendra compte de la nature des principes actifs entrant dans l'association.

Ainsi, lorsque l'inhibiteur sélectif du facteur Xa est un oligosaccharide, celui-ci est de préférence utilisé sous forme de sel d'addition dans la composition, par exemple sous forme de sel sodique.

Normalement, les oligosaccahrides sous forme de leurs sels d'addition avec des acides pharmaceutiquement acceptables, ne sont pas chimiquement incompatibles avec les antiagrégants plaquettaires non salifiés. Toutefois, certains de ces derniers sont également utilisés sous forme de sels d'addition acides. En tout état de cause, il est préférable de maintenir les principes actifs séparés selon des techniques bien connues de la littérature.

Les compositions pharmaceutiques de la présente invention sont particulièrement indiquées dans le traitement ou la prophylaxie des états pathologiques tels que les troubles du système cardiovasculaire et cérébrovasculaire comme les troubles tromboemboliques associés à l'athérosclérose ou au diabète tels l'angine instable, l'attaque cérébrale, la resténose après l'angioplastie, l'endartérectomie ou pose de prothèses endovasculaires métalliques, ou comme les troubles thromboemboliques associés à la rethrombose après thrombolyse, à l'infarctus, à la démence d'origine ischémique, aux maladies artérielles périphériques, à l'hémodialyse, aux fibrillations auriculaires, ou encore, lors de l'utilisation de prothèses vasculaires de pontages aorto-coronariens ou vis à vis de l'angor stable ou instable, ou encore. *vis à vis* de patients traités par une procédure de revascularisation à risques de thrombose incluant les angioplasties percutanées, les prothèses endovasculaires, les prothèses vasculaires, les pontages aorto-coronariens.

Les associations selon l'invention peuvent être formulés dans des compositions pharmaceutiques pour l'administration aux mammifères, y compris l'homme, pour le traitement des maladies susdites.

Les associations selon l'invention peuvent être utilisés à des doses journalières de l'inhibiteur sélectif du facteur Xa ou de l'antiagrégant plaquettaire de 0,1 à 100 mg par kilo de poids corporel du mammifère à traiter.

Chez l'être humain, la dose peut varier pour chacun des composants de 1 à 500 mg par jour, selon l'age du sujet à traiter ou le type de traitement : prophylactique ou curatif. De préférence, le pentasaccharide est administré à des doses comprises entre 0,30 mg et 30 mg par patient et par jour.

Dans les compositions pharmaceutiques de la présente invention, les principes actifs sont généralement formulés en unités de dosage contenant de 0,1 à 50 mg dudit principe actif par unité de dosage.

Les compositions de l'invention sont réalisées de façon à pouvoir être administrées par la voie digestive ou parentérale et sont présentées sous des formes diverses, telles que par exemple, des solutions injectables ou buvables, dragées, comprimés ou gélules. Les solutions injectables sont les formes pharmaceutiques préférées.

Dans les composition pharmaceutiques de la présente invention pour l'administration orale, sublinguale, sous-cutanée, intramusculaire, intraveineuse, transdermique, transmuqueuse, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique -ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration transmuqueuse, les principes actifs peuvent être formulés en présence d'un promoteur tel qu'un sel biliaire, d'un polymère hydrophile tel que par exemple l'hydropropylcellulose, l'hydroxypropylméthylcellulose, l'hydroxyéthylcellulose, l'éthylcellulose, la carboxyméthylcellulose, le dextran, la polyvinylpyrrolidone, les pectines, les amidons, la gélatine, la caséine, les acides acryliques, les esters acryliques et leurs copolymères, les polymères ou copolymères de vinyle, les alcools vinyliques, les alcoxypolymères, les polymères d'oxyde de polyéthylène, les polyéthers ou leur mélange.

Les principes actifs peuvent être formulés également sous forme de microcapsules, éventuellement avec un ou plusieurs supports ou additifs.

Les principes actifs peuvent être également présentés sous forme de complexe avec une cyclodextrine, par exemple α, β ou γ cyclodextrine. 2-hydroxypropyl-β-cyclodextrine ou méthyl-β-cyclodextrine.

Un des principe actif, par exemple l'oligosaccharide inhibiteur du facteur Xa, peut également être libéré par un ballonnet le contenant ou par un extenseur endovasculaire introduit dans les vaisseaux sanguins. L'efficacité pharmacologique du principe actif n'est ainsi pas affectée.

De préférence, l'inhibiteur sélectif du facteur Xa est administré par voie intraveineuse ou sous cutanée.

Dans les associations thérapeutiques selon la présente invention, les formes pharmaceutiques contiennent, de préférence, 8 à 30 mg de l'inhibiteur sélectif du facteur Xa et 10 à 200 mg du composé antiagrégant plaquettaire.

L'association de 15 à 25 mg d'inhibiteur sélectif du facteur Xa et de 10 à 30 mg de l'antiagrégant plaquettaire est particulièrement avantageuse. Mieux encore, les formes pharmaceutiques de la présente invention comprennent 20 mg d'un pentasaccharide inhibiteur sélectif du facteur Xa et 20 mg d'un antiagrégant plaquettaire.

## Revendications

1. Utilisation d'un oligosaccharide inhibiteur indirect du facteur Xa agissant *via* l'antithrombine III, en combinaison avec un ou plusieurs composés à activité antiagrégante plaquettaire antagoniste de la glycoprotéine IIb/IIIa choisis parmi
- le N-(1-éthoxycarbonylméthyl-pipéridin-4-yl)-N-{4-[4-{(N-éthoxycarbonylimino) (amino)méthyl}-phényl]-thiazol-2-yl}-3-aminopropionate d'éthyle,
- l'acide N-(1-carboxyméthyl-pipéridin-4-yl)-N-{4-[4-{(amino)(imino)méthyl}phényl]-thiazol-2-yl}-3-aminopropionique, et leurs sels pharmaceutiquement acceptables, pour la préparation de médicaments destinés à prévenir et à traiter les maladies thromboemboliques artérielles.

2. Utilisation selon la revendication 1, **caractérisée en ce que** l'inhibiteur indirect du facteur Xa est le méthyl O-(2-déoxy-2-sulfoamino-6-O-sutfo-α-D-glucopyranosyl)-(1→4)-O-(acide β-D-glucopyranosyluronique)-(1→4)-O-(2-déoxy-2-sulfoamino-3,6-di-O-sulfo-α-D-glucvpyranosyt-(1→4)-O-(acide 2-O-sulfo-α-L-idopyranosyluronique)-(1→4)-2-déoxy-2-sulfoamino-6-O-sulpho-α-D-glucopyranoside dont l'anion a la structure (B) 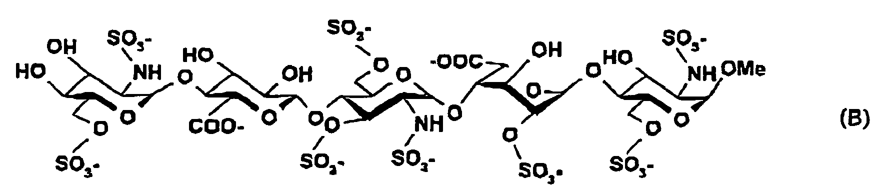 ou l'un de ses sels pharmaceutiquement acceptables.

3. Utilisation selon l'une des revendications 1 ou 2 **caractérisée en ce que** l'antagoniste de la glycoprotéine IIb/IIIa est le N-(1-èthoxycarbonylméthyl-pipéridin-4-yl)-N-{4-[4-{(N-éthoxycarbonylimino)(amino)méthyl}-phényl]-thiazol-2-yl}-3-aminopropionate d'éthyle ou le trichlorhydrate de l'acide N-(7-carbvxyméthylpipéridin-4-yl)-N-{4-[4{(amino)(imino)méthyl}phényl]-thiazol-2-yl}-3-aminopropionique.

4. Compositions pharmaceutiques contenant un oligosaccharide inhibiteur indirect du facteur Xa agissant via l'antithrombine III, en combinaison avec un ou plusieurs composés à activité antiagrégante plaquettaire antagoniste de la glycoprotéine IIb/IIIa choisis parmi
- le N-(1-éthoxycarbonylméthyl-pipéridin-4-yl)-N-(4-[4-{(N-êthoxycarbonylimino) (amino)méthyl}-phényl]-thiazol-2-yl}-3-aminopropionate d'éthyle,
- l'acide N-(1-carboxyméthyl-pipéridin-4-yl)-N-{4-[4-{(amino)(lmino)méthyl}phényl]-thiazol-2-yl}-3-aminopropivnique, et leurs sels pharmaceutiquement acceptables, et, éventuellement un ou plusieurs véhicules pharmaceutiquement acceptables.

5. Compositions pharmaceutiques selon la revendication 4 comprenant le méthyl O-(2-dévxy-2-suifoamino-6-O-sulfo-α-D-glucopyranosyl)(1→4)-O-(acide β-D-glucopyranosyturonique)-(1→4)-O-(2-déoxy-2-sulfoamino-3,6-di-O-sulfo-α-D-glucopyranosyl-(1→4)-O-(acide 2-O-sulfo-α-L-idopyranosyluronique)-(1→4)-2-déoxy-2-sulfoamino-6-O-sulpho-α-D-glucopyranoside dont l'anion a la structure (B) 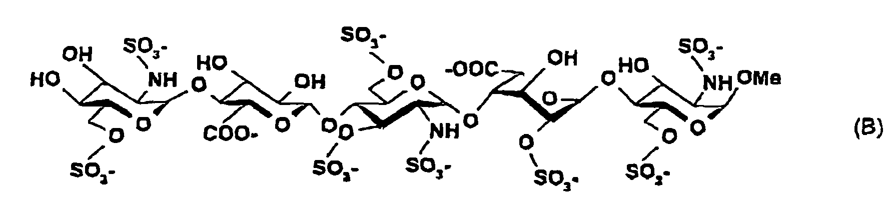 ou l'un de ses sels pharmaceutiquement acceptables, en tant qu'inhibiteur sélectif indirect du facteur Xa.

6. Utilisation selon la revendication 1, lesdits médicaments étant actifs *vis à vis* d'états pathologiques tels que les troubles du système cardiovasculaire et cérébrovasculaire comme les troubles thromboemboliques associés à l'athérosclérose ou au diabète tels l'angine instable, l'attaque cérébrale, la resténose après angioplastie, l'endartérectomie ou pose de prothèses endovasculaires métalliques ou comme les troubles thromboemboliques associés à la rethrombose après thrombolyse, à l'infarctus, à la démence d'origine ischémique, aux maladies artérielles périphériques, à l'hémodialyse, aux fibrillations auriculaires, ou encore, lors de l'utilisation de prothèses vasculaires, ou de pontages aorto coronariens, ou, *vis à vis* de l'angor stable ou instable, ou encore, *vis à vis* de patients traités par une procédure de revascularisation à risques de thrombose incluant les angioplasties percutanées, les prothèses endovasculaires, les prothèses vasculaires, les pontages aorto-coronariens.

## Claims

1. Use of an oligosaccharide that is an indirect inhibitor of factor Xa acting via antithrombin III, in combination with one or more compounds with anti-platelet aggregation activity antagonizing glycoprotein IIb/IIIa, selected from
- ethyl N-(1-ethoxycarbonylmethylpiperidin-4-yl)-N-{4-[4-{(N-ethoxycarbonylimino)(amino)methyl}phenyl]-thiazol-2-yl}-3-aminopropionate,
- N-(1-carboxymethylpiperidin-4-yl)-N-{4-[4-{(amino)-(imino)methyl}phenyl]thiazol-2-yl}-3-aminopropionic acid, and their pharmaceutically acceptable salts for the preparation of medicines intended to prevent and to treat thromboembolic arterial diseases.

2. Use according to Claim 1, **characterized in that** the indirect inhibitor of factor Xa is methyl O-(2-deoxy-2-sulphoamino-6-O-sulfo-α-D-glucopyranosyl)-(1→4)-O-(β-D-glucopyranosyluronic acid)-(1→4)-O-(2-deoxy-2-sulphoamino-3,6-di-O-sulpho-α-D-glucopyranosyl-(1→4)-O-(2-O-sulpho-α-L-idopyranosyluronic acid)-(1→4)-2-deoxy-2-sulphoamino-6-O-sulpho-α-D-glucopyranoside whose anion has the structure (B) 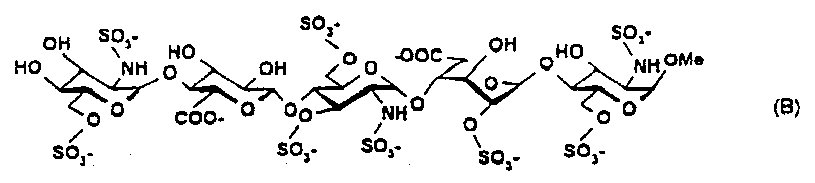 or one of its pharmaceutically acceptable salts.

3. Use according to either of Claims 1 and 2, **characterized in that** the antagonist of glycoprotein IIb/IIIa is ethyl N-(1-ethoxycarbonylmethylpiperidin-4-yl)-N-{4-[4-{(N-ethoxycarbonylimino)(amino)methyl}-phenyl]thiazol-2-yl}-3-aminopropionate or N-(1-carboxymethylpiperidin-4-yl)-N-{4-[4-{(amino)-(imino)methyl}phenyl]thiazol-2-yl}-3-aminopropionic acid trihydrochloride.

4. Pharmaceutical compositions containing an oligosaccharide that is an indirect inhibitor of factor Xa acting via antithrombin III, in combination with one or more compounds with anti-platelet aggregation activity antagonizing glycoprotein IIb/IIIa, selected from
- ethyl N-(1-ethoxycarbonylmethylpiperidin-4-yl)-N-{4-[4-{(N-ethoxycarbonylimino)(amino)methyl}phenyl]-thiazol-2-yl}-3-aminopropionate,
- N-(1-carboxymethylpiperidin-4-yl)-N-{4-[4-{(amino)-(imino)methyl}phenyl]thiazol-2-yl}-3-aminopropionic acid, and their pharmaceutically acceptable salts and optionally one or more pharmaceutically acceptable vehicles.

5. Pharmaceutical compositions according to Claim 4, comprising methyl O-(2-deoxy-2-sulphoamino-6-O-sulfo-α-D-glucopyranosyl)-(1→4)-O-(β-D-glucopyranosyluronic acid-(1→4)-O-(2-deoxy-2-sulphoamino-3,6-di-O-sulpho-α-D-glucopyranosyl-(1→4)-O-(2-O-sulpho-α-L-idopyranosyluronic acid)-(1→4)-2-deoxy-2-sulphoamino-6-O-sulpho-α,-D-glucopyranoside whose anion has the structure (B) 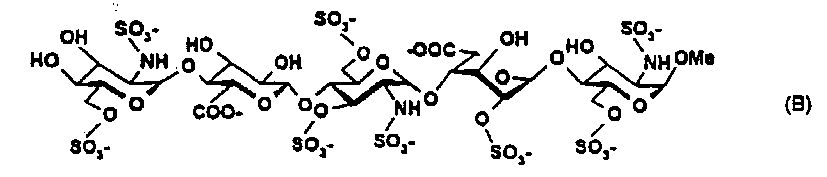 or one of its pharmaceutically acceptable salts, as indirect selective inhibitor of factor Xa.

6. Use according to Claim 1, the said medicaments being active for pathological conditions such as disorders of the cardiovascular or cerebrovascular system such as thromboembolic disorders associated with atherosclerosis or with diabetes such as unstable angina, cerebral attack, restenosis following angioplasty, endarterectomy or fitting of metallic endovascular prostheses, or such as thromboembolic disorders associated with rethrombosis following thrombolysis, with infarction, with dementia of ischaemic origin, with peripheral arterial diseases, with haemodialysis, with atrial fibrillations, or during the use of vascular prostheses, of aortocoronary bypasses or for stable or unstable angina, or for patients treated by a revascularization procedure at the risk of thrombosis including percutaneous angioplasties, endovascular prostheses, vascular prostheses, aortocoronary bypasses.

## Patentansprüche

1. Verwendung eines Oligosaccharids, das ein über Antithrombin III wirkender, indirekter Inhibitor von Faktor Xa ist, in Kombination mit einer oder mehreren Verbindungen, die eine die Plättchenaggregation hemmende Glykoprotein-IIb/IIIa-Antagonistenaktivität besitzen, ausgewählt aus
- Ethyl-N-(1-ethoxycarbonylmethylpiperidin-4-yl)-N-{4-[4-{(N-ethoxycarbonylimino)-(amino)-methyl}-phenyl]-thiazol-2-yl}-3-aminopropionat,
- N-(1-Carboxymethylpiperidin-4-yl)-N-{4-[4-{(amino)-(imino)-methyl}-phenyl]-thiazol-2-yl}-3-aminopropionsäure und ihren pharmazeutisch annehmbaren Salzen, zur Herstellung von Medikamenten, die dazu bestimmt sind, arterielle thromboembolische Erkrankungen zu verhindern und zu behandeln.

2. Verwendung nach Anspruch 1, **dadurch gekennzeichnet, dass** der indirekte Faktor-Xa-Inhibitor Methyl-O-(2-desoxy-2-sulfoamino-6-O-sulfo-α-D-glucopyranosyl)-(1→4)-O-(β-D-glucopyranosyluronsäure)-(1→4)-O-(2-desoxy-2-sulfoamino-3,6-di-O-sulfo-α-D-glucopyranosyl-(1→4)-O-(2-O-sulfo-α-L-idopyranosyluronsäure)-(1→4)-2-desoxy-2-sulfoamino-6-O-sulfo-α-D-glucopyranosid, dessen Anion die Struktur (B) hat 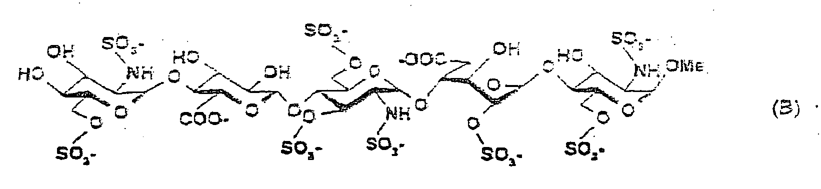 oder eines seiner pharmazeutisch annehmbaren Salze ist.

3. Verwendung nach einem der Ansprüche 1 oder 2, **dadurch gekennzeichnet, dass** der Glykoprotein-IIb/IIIa-Antagonist Ethyl-N-(1-ethoxycarbonylmethylpiperidin-4-yl)-N-{4-[4-{(N-ethoxycarbonylimino)(amino)-methyl}-phenyl]-thiazol-2-yl}-3-aminopropionat oder N-(1-Carboxymethylpiperidin-4-yl)-N-{4-[4-{(amino)(imino)-methyl}-phenyl]-thiazol-2-yl}-3-aminopropionsäuretrihydrochlorid ist.

4. Pharmazeutische Zusammensetzungen, die ein Oligosaccharid, das ein über Antithrombin III wirkender, indirekter Inhibitor von Faktor Xa ist, in Kombination mit einer oder mehreren Verbindungen, die eine die Plättchenaggregation hemmende Glykoprotein-IIb/IIIa-Antagonistenaktivität besitzen, ausgewählt aus
- Ethyl-N-(1-ethoxycarbonylmethylpiperidin-4-yl)-N-{4-[4-{(N-ethoxycarbonylimino)-(amino)-methyl}-phenyl]-thiazol-2-yl}-3-aminopropionat,
- N-(1-Carboxymethylpiperidin-4-yl)-N-{4-[4-{(amino)-(imino)-methyl}-phenyl]-thiazol-2-yl}-3-aminopropionsäure und ihren pharmazeutisch annehmbaren Salzen, und gegebenenfalls ein oder mehrere pharmazeutisch annehmbare Vehikel enthalten.

5. Pharmazeutische Zusammensetzungen nach Anspruch 4, die Methyl-O-(2-desoxy-2-sulfoamino-6-O-sulfo-α-D-glucopyranosyl)-(1→4)-O-(β-D-glucopyranosyluronsäure)-(1→4)-O-(2-desoxy-2-sulfoamino-3,6-di-O-sulfo-α-D-glucopyranosyl-(1→4)-O-(2-O-sulfo-α-L-idopyranosyluronsäure)-(1→4)-2-desoxy-2-sulfoamino-6-O-sulfo-α-D-glucopyranosid, dessen Anion die Struktur (B) hat 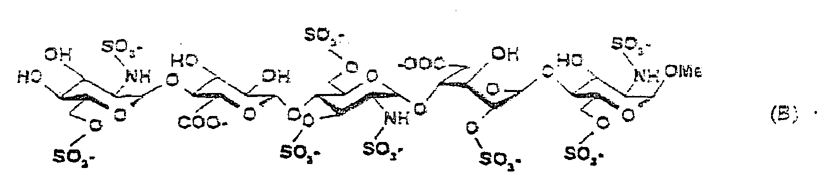 oder eines seiner pharmazeutisch annehmbaren Salze als selektiven indirekten Inhibitor von Faktor Xa enthalten.

6. Verwendung nach Anspruch 1, wobei diese Medikamente gegen pathologische Zustände wirksam sind, wie Störungen der kardiovaskulären und des zerebrovaskulären Systems, wie thromboembolische Störungen in Verbindung mit Atherosklerose oder Diabetes, wie instabile Angina, Hirnschlag, Restenose nach Angioplastie, Endarterektomie oder Einsetzen metallischer endovaskulärer Prothesen, oder wie thromboembolische Störungen in Verbindung mit Rethrombose nach Thrombolyse, Infarkt, Demenz ischämischer Ursache, Erkrankungen der peripheren Arterien, Hämodialyse, Vorhofflimmern, oder auch bei Verwendung von Gefäßprothesen oder aorto-koronaren Bypässen oder gegen stabile oder instabile Angina pectoris oder auch bei einem Thromboserisiko unterliegenden Patienten, die durch ein Revaskularisierungsverfahren, einschließlich perkutaner Angioplastien, endovaskulärer Prothesen, aorto-koronaren Bypässen, behandelt wurden.
